# FASCICULE DE BREVET EUROPEEN

(11) **EP 2 903 738 B1**
(45) Date de publication et mention de la délivrance du brevet: **14.09.2016**
(21) Numéro de dépôt: 13774166.6
(22) Date de dépôt: 08.10.2013
(51) Int. Cl.: C12Q 1/68, B01L 3/00, B01L 7/00

(54) **PROCÉDÉ MICROFLUIDIQUE DE TRAITEMENT ET D'ANALYSE D'UNE SOLUTION CONTENANT UN MATÉRIEL BIOLOGIQUE, ET CIRCUIT MICROFLUIDIQUE CORRESPONDANT**
MIKROFLUIDISCHES VERFAHREN ZUR BEHANDLUNG UND ANALYSE EINER LÖSUNG MIT EINEM BIOLOGISCHEM MATERIAL UND ENTSPRECHENDE MIKROFLUIDISCHE SCHALTUNG
MICROFLUIDIC PROCESS FOR TREATING AND ANALYSING A SOLUTION CONTAINING A BIOLOGICAL MATERIAL, AND CORRESPONDING MICROFLUIDIC CIRCUIT

(30) Priorité: 08.10.2012 FR 1259566
(43) Date de publication de la demande: 12.08.2015
(73) Titulaire: Ecole Polytechnique, 91120 Palaiseau (FR); CENTRE NATIONAL DE LA RECHERCHE SCIENTIFIQUE - CNRS, 75016 Paris 16 (FR)
(72) Inventeur: BAROUD, Charles, F-75013 Paris (FR); DANGLA, Rémi, F-75009 Paris (FR); ABBYAD, Paul, Santa Clara, California 95050 (US); TURKCAN, Silvan, F-75013 Paris (FR)
(74) Mandataire: Icosa
(86) Numéro de dépôt international: PCT/EP2013/070966
(87) Numéro de publication internationale: WO 2014/056930

(56) Documents cités:
- WO-A1-2011/121220
- DE-A1-102005 037 401
- US-A1- 2010 190 263
- BEER N R ET AL: "On-chip, real-time, single-copy polymerase chain reaction in picoliter droplets", ANALYTICAL CHEMISTRY, AMERICAN CHEMICAL SOCIETY, US, vol. 79, no. 22, 15 novembre 2007 (2007-11-15), pages 8471-8475, XP002575962, ISSN: 0003-2700, DOI: 10.1021/AC701809W [extrait le 2007-10-11]
- RALF SEEMANN ET AL: "Droplet based microfluidics;Droplet based microfluidics", REPORTS ON PROGRESS IN PHYSICS, INSTITUTE OF PHYSICS PUBLISHING, BRISTOL, GB, vol. 75, no. 1, 22 décembre 2011 (2011-12-22), page 16601, XP020216246, ISSN: 0034-4885, DOI: 10.1088/0034-4885/75/1/016601

## Description

### 1. Domaine de l'invention

La présente invention concerne un procédé de traitement et d'analyse d'une solution contenant un matériel biologique, mettant en oeuvre une méthode microfluidique dans laquelle la solution est divisée en une pluralité de gouttes.

L'invention concerne également un circuit microfluidique, permettant la manipulation de très petites quantités de fluides, permettant notamment de mettre en oeuvre un tel procédé.

### 2. Art antérieur

On connaît, notamment des documents FR-2873171, FR-2901717, WO-2011/121220 et WO-2011/039475, au nom des demanderesses, des procédés microfluidiques permettant la fabrication et la manipulation, dans des circuits microfluidiques adaptés, de gouttes d'un premier fluide placées dans un second fluide, appelé fluide porteur. Le premier fluide est généralement une solution aqueuse, divisée en gouttes d'un volume de l'ordre de 10 à 100 µm³. Le fluide porteur est généralement de l'huile, pouvant être additionnée d'un produit surfactant permettant d'éviter la fusion spontanée des gouttes de fluide manipulées, si elles entrent en contact.

Il a été proposé d'utiliser de tels procédés microfluidiques pour appliquer des traitements à une solution contenant un matériel biologique, suivis d'analyses de la solution traitée. Il a été notamment envisagé d'utiliser de telles techniques pour mise en oeuvre de méthodes d'amplification en chaîne par polymérase (couramment désignée par l'acronyme « PCR » pour l'expression anglaise « Polymerase Chain Reaction ») qui permettent de copier en grand nombre une séquence d'acide nucléique, tel que de l'ADN (acronyme pour «Acide Désoxyribonucléique ») ou de l'ARN (acronyme pour « Acide Ribonucléique »). Pour réaliser cette amplification, on prépare une solution contenant une faible quantité d'acide nucléique, que l'on soumet à un traitement thermique appelé thermocyclage, consistant en des variations cycliques de température. Ces variations de température permettent des duplications forcées des molécules d'acide nucléique présentes dans la solution. Il est ainsi possible d'augmenter très fortement la concentration de l'acide nucléique dans la solution.

Ces méthodes d'amplification en chaîne par polymérase, qui peuvent se décliner en un grand nombre de variantes, sont bien connues de l'homme du métier de la biologie moléculaire. Les méthodes d'amplification en chaîne par polymérase utilisant les procédés microfluidiques pour diviser la solution contenant l'acide nucléique en de nombreuses portions de faible volume, avant l'amplification, sont également connues en elles-mêmes de l'homme du métier, et sont couramment appelées « PCR digitales ».

On connaît notamment du document WO 2010/036352 un tel procédé de PCR digitale. Selon ce procédé, on utilise un écoulement, ou flux, de fluide porteur, pour diviser la solution contenant l'acide nucléique en une grande quantité de gouttes. La concentration de l'acide nucléique dans la solution est choisie pour que, statistiquement, un faible nombre de gouttes contienne une molécule de l'acide nucléique recherché. Les gouttes sont placées dans un récipient pour subir un thermocyclage, permettant l'amplification en chaîne par polymérase de l'acide nucléique. Elles sont ensuite introduites dans un canal pour être analysées optiquement, les unes après les autres, afin de détecter celles qui contenaient, avant le thermocyclage, au moins une occurrence de l'acide nucléique, et qui contiennent après ce thermocyclage une grande quantité de cet acide nucléique.

Ce procédé nécessite l'utilisation d'équipements nombreux et couteux pour, d'une part, produire les gouttes, puis pour assurer le thermocyclage, et enfin pour analyser les gouttes après leur thermocyclage. Par ailleurs, ces opérations successives sont longues et nécessitent des compétences importantes. Une amplification en chaine par polymérase selon ce procédé est en conséquence longue, couteuse, et ne peut être réalisée que par des opérateurs spécialement formés.

Par ailleurs, lorsque l'on utilise un flux de fluide porteur pour produire des gouttes à partir de l'échantillon de solution contenant l'acide nucléique, les premières gouttes, produites au cours d'une phase transitoire, présentent des dimensions qui ne sont pas appropriées. Seules les gouttes produites lors d'une seconde phase, qui présentent des dimensions plus homogènes, peuvent être exploitées pour l'amplification en chaine par polymérase. Ce procédé implique donc la perte d'une part significative de l'échantillon initial de solution contenant l'acide nucléique. D'autres pertes d'une partie de cette solution sont générées par les transferts nécessaires entre différents récipients. Ce procédé peut donc engendrer des pertes importantes de l'échantillon, pouvant être de l'ordre de 25 %. Les échantillons biologiques étant parfois extrêmement rares et couteux, une telle perte représente un inconvénient important.

On connaît également, de l'article « 1-Million droplet array with wide-field flaforescence imaging for digital PCR », de Hatch, Fisher, Tovar, Hsieh Lin, Pentoney, Yan et Lee (Lab Chip, 2011, 11, 3838*)* un autre procédé de PCR digitale par gouttes, dans lequel les gouttes de solution contenant l'acide nucléique sont créées par huit divisions successives d'une goutte en deux gouttes de taille égale. Ces divisions successives permettent de créer, à partir d'une goutte initiale, 256 gouttes de taille égale qui sont poussées dans un canal plat de grande largeur. Après la production d'un grand nombre de ces gouttes, une portion significative du canal peut être remplie. Le canal et les gouttes qu'il contient peuvent alors être soumis au thermocyclage permettant l'amplification en chaine par polymérase. Par la suite, l'analyse des différentes gouttes peut être faite directement, sans les sortir du canal, par observation optique des gouttes à travers une paroi transparente du canal.

Ce procédé présente également certains inconvénients. Ainsi, il impose de disposer d'une goutte initiale d'une taille bien définie, qui puisse être divisée en gouttes de taille adaptée au traitement et à la mesure ultérieurs. Or, la méthode utilisée pour la production des gouttes initiales, par divusion d'un flux de solution sous l'action d'un flux de fluide porteur, suppose une phase transitoire au début de la production des gouttes, au cours de laquelle les écoulements de la solution contenant l'acide nucléique et du fluide porteur doivent s'équilibrer. Les gouttes formées au cours de cette phase transitoire présentent donc une taille non adaptée. Les divisions successives de ces gouttes initiales entrainent l'introduction dans le canal d'un grand nombre de gouttes de taille inadaptée, qui ne peuvent pas être validement analysées. En conséquence, seule une partie de l'échantillon de fluide biologique peut être analysée, une autre part, représentant environ 10 % de l'échantillon, étant perdue.

Par ailleurs, les gouttes ne pouvant être produites et divisées que sous l'action d'un flux de fluide porteur, une grande quantité de ce fluide porteur est introduite dans le canal en même temps que les gouttes. En conséquence, la concentration des gouttes dans le fluide porteur, dans ce canal, n'est pas optimale.

Enfin, ce procédé, nécessitant l'équilibrage d'un flux de solution contenant l'acide nucléique et d'un flux de fluide biologique, est relativement complexe à mettre en oeuvre et nécessite des compétences particulières. En effet, sans une mise en oeuvre rigoureuse du procédé, les gouttes produites peuvent présenter des tailles non homogènes, ce qui est préjudiciable à l'analyse.

### 3. Objectifs de l'invention

La présente invention a pour objectif de palier ces inconvénients de l'art antérieur.

En particulier, la présente invention a pour objectif de proposer un procédé de traitement et d'analyse d'une solution contenant un matériel biologique, mettant en oeuvre une méthode microfluidique dans laquelle la solution est divisée en une pluralité de gouttes, qui soit plus rapide à mettre en oeuvre que les procédés de l'art antérieur, plus efficace, plus simple et moins couteuse, qui nécessite une formation moindre des opérateurs devant mettre en oeuvre le procédé, et qui permette de traiter et d'analyser utilement une proportion plus importante du matériel biologique consommé.

Un autre objectif de la présente invention est de fournir un circuit microfluidique permettant la mise en oeuvre d'un tel procédé.

L'invention a notamment pour objectif, selon au moins un de ses modes de réalisation, de fournir un tel procédé, et le circuit microfluidique permettant de le mettre en oeuvre, qui permette de réaliser une PCR digitale par gouttes plus simple, plus efficace et moins couteuse que les procédés de l'art antérieur.

### 4. Exposé de l'invention

Ces objectifs, ainsi que d'autres qui apparaîtront plus clairement par la suite, sont atteints à l'aide d'un procédé microfluidique de traitement et d'analyse d'une solution contenant un matériel biologique, le procédé comprenant, selon l'invention, les étapes suivantes :
- introduction de la solution dans des microcanaux d'un circuit microfluidique ;
- détachement de gouttes de la solution dans un fluide porteur, causé par l'écartement des parois des microcanaux, couplé avec les effets de la tension de surface de la solution ;
- déplacement d'au moins une partie des gouttes dans le fluide porteur vers au moins une zone de stockage de gouttes dans le circuit microfluidique, causé par l'écartement des parois des microcanaux, couplé avec les effets de la tension de surface des gouttes ;
- application d'un traitement aux gouttes situées dans la ou les zones de stockage ;
- analyse des gouttes situées dans la ou les zones de stockage.

Ce procédé permet avantageusement que la réaction générée par le traitement se déroule de façon indépendante dans chacune des gouttes. Il peut être mis en oeuvre particulièrement facilement, dans un seul circuit microfluidique dans lequel sont réalisées les différentes étapes. Par ailleurs, les gouttes peuvent être fabriquées et transportées indépendamment de la présence ou non d'un écoulement du fluide porteur. La taille des gouttes, notamment, ne dépend pas fortement d'un mouvement du fluide porteur, et est homogène dès le début de leur formation. Il est ainsi possible que la totalité, ou la quasi-totalité de l'échantillon consommé subisse le traitement et soit analysé.

Pour cela, les microcanaux du circuit microfluidique sont configurés pour que la solution y circule entre des parois qui s'écartent les unes des autres, en causant une variation du confinement de la solution. L'écartement de chaque paroi peut être progressif (parois en pentes) ou abrupt (marche). La tension de surface de la solution, c'est à dire la tension interfaciale entre la solution et le fluide porteur avec lequel elle est en contact, impose au flux de solution une forme tenant compte de ce confinement variable, qui aboutit à la séparation de gouttes.

Cette méthode de séparation de gouttes, dans laquelle la tension de surface de la solution est utilisée pour causer le détachement de la goutte, se distingue donc radicalement des méthodes nécessitant un flux de fluide porteur pour créer une goutte par cisaillement de la solution, en s'opposant à la tension de surface de la solution qui tend au contraire à rassembler la solution. Elle présente également l'avantage de ne pas exiger d'équilibrage d'un flux de fluide porteur avec le flux de solution, ce qui simplifie le procédé.

Le déplacement des gouttes est également causé par l'écartement des parois couplé avec les effets de la tension de surface des gouttes. Il peut être causé directement, une goutte se déplaçant entre des parois s'écartant, sous l'effet de sa tension de surface, ou indirectement, la goutte étant poussée par une autre goutte, qui elle-même se se déplace entre les parois s'écartant, sous l'effet de sa tension de surface.

Enfin, les gouttes sont maintenues, après leur formation et leur déplacement, dans au moins une zone de stockage, qui est une zone dans laquelle elle peuvent pénétrer, mais dont elle ne peuvent pas sortir sans une intervention extérieure (par exemple un flux de fluide porteur leur conférant une énergie suffisante pour sortir). Elles peuvent ainsi très facilement être soumises à un traitement et être analysées.

Avantageusement, le fluide porteur dans lequel les gouttes sont détachées et déplacées est sensiblement statique.

La fabrication et le déplacement des gouttes sont ainsi ainsi plus fiables, dans la mesure où ils sont définis uniquement par la configuration des parois des microcanaux, sans être perturbés par un écoulement de fluide porteur. Bien entendu, le fluide porteur, bien que sensiblement statique, subit de légères perturbations causées par le déplacement des gouttes.

Selon un mode de réalisation avantageux de l'invention, le traitement appliqué aux gouttes comprend des variations de la température des gouttes.

Préférentiellement, dans ce cas, les variations de température sont appliquées à l'ensemble du circuit microfluidique contenant les gouttes. Elles peuvent aussi être appliquées à des sous-régions ou bien à des gouttes individuelles, par exemple successivement les unes après les autres.

Ces variations de températures peuvent en effet être facilement appliqué au circuit microfluidique et à l'ensemble des gouttes qu'il contient. Les transferts des gouttes d'un récipient vers un autre sont ainsi évités.

Les variations de température, ou thermocyclage, peuvent par exemple permettre la réalisation d'une amplification en chaîne par polymérase. D'autres traitements peuvent également être appliqués, comme par exemple une incubation, consistant à maintenir les gouttes, pendant un temps suffisamment long, à des conditions de température permettant qu'une réaction se produise.

Préférentiellement, l'analyse des gouttes est une analyse optique.

Cette analyse peut être réalisée facilement, à travers les parois du circuit microfluidique, sans qu'aucun transfert des gouttes ne soit nécessaire.

Selon un mode de réalisation avantageux de l'invention, au moins une des zones de stockage est constituée par une zone dans laquelle les gouttes présentent une énergie de surface plus faible que dans les zones voisines.

Ainsi, la configuration des microcanaux du circuit microfluidique permet que les gouttes soient maintenues dans la zone de stockage, qui peut également être appellée zone de piégeage, sous l'effet de leur tension de surface. Elles sont donc maintenue efficacement dans cette zone de stockage, indépendamment d'un éventuel flux de fluide porteur, tant que ce flux de fluide porteur ou une autre action extérieure, par exemple la poussée d'une autre goutte, ne leur confère une énergie suffisante pour élever son énergie de surface en passant dans une zone entourant la zone de stockage.

De façon avantageuse, le matériel biologique contenu dans la solution comprend au moins un acide nucléique, et le traitement appliqué aux gouttes est une amplification en chaîne par polymérase, permettant d'augmenter la concentration d'au moins une séquence dudit acide nucléique.

Le procédé selon l'invention permet ainsi de réaliser une amplification en chaîne par polymérase digitale par gouttes, qui est plus simple et plus efficace que celles mises en oeuvre dans l'art antérieur.

L'invention concerne également un circuit microfluidique, dans lequel sont définis des microcanaux pouvant contenir des fluides, le circuit comprenant au moins un dispositif de formation de gouttes d'une solution dans un fluide porteur, et au moins une zone de stockage des gouttes ainsi produites. Selon l'invention, les dispositifs de formation des gouttes comprennent des portions de paroi des microcanaux, s'écartant les unes des autres de façon à détacher une goutte de la solution sous l'effet de la tension de surface de la solution, et le circuit microfluidique comprend des moyens de guidage des gouttes comprenant des portions de paroi des microcanaux, s'écartant les unes des autres de façon à déplacer les gouttes vers la zone de stockage sous l'effet de la tension des gouttes.

Ce circuit permet notamment la mise en oeuvre du procédé décrit ci-dessus, de façon particulièrement facile. Aucun flux de fluide porteur n'est en effet nécessaire dans ce circuit, la seule introduction de la solution dans le circuit entrainant automatiquement sa division en gouttes et le déplacement de ces gouttes vers la zone de stockage où elles peuvent être traitées et analysées.

Préférentiellement, au moins une des zones de stockage est constituée par une zone d'un microcanal dans laquelle les parois dudit microcanal sont plus éloignées les unes des autres que dans les zones voisines.

Cette zone de stockage peut par exemple être définie dans une chambre, dans la quelle les gouttes ne sont confinées que par une paroi supérieure et une paroi inférieure. Une zone de cette chambre dans laquelle ces deux parois sont plus éloignées permet aux gouttes d'y être moins confinées. Cette zone retient alors les gouttes et constitue une zone de stockage.

Selon un mode de réalisation avantageux, le circuit microfluidique contient au moins deux zones de stockage distinctes.

Il est ainsi possible de traiter et d'analyser simultanément plusieurs groupes de gouttes distincts.

De façon avantageuse, le circuit microfluidique comprend au moins deux dispositif de formation de gouttes, permettant chacun de former des gouttes de volumes différents.

Ainsi, le circuit permet de traiter et d'analyser simultanément des gouttes de plusieurs tailles.

Avantageusement, dans ce cas, les moyens de guidage des gouttes sont configurés de façon à guider les gouttes de volumes différents vers des zones de stockage distinctes.

Selon un mode de réalisation avantageux, au moins une desdites zones de stockage est configurée de manière à ne pouvoir recevoir qu'une goutte.

Dans ce cas, chaque goutte peut être maintenue dans sa zone de stockage, ou de piégeage individuelle. Cela permet un meilleur positionnement de gouttes, permettant notamment de faciliter leur analyse. Les gouttes n'étant, dans ce cas, pas en contact les unes avec les autres pendant leur traitement et leur analyse, elles ne risquent pas de fusionner. Il est donc possible, dans ce cas, de réduire les propriétés surfactantes du fluide porteur sans inconvénient.

Selon un autre mode de réalisation avantageux, au moins une des zones de stockage est configurée de manière à recevoir des gouttes dans un même plan.

Un tel mode de réalisation permet de faciliter l'analyse des gouttes.

Selon un autre mode de réalisation avantageux, au moins une des zones de stockage est configurée de manière à répartir sur au moins deux plans superposés les gouttes qu'elle contient.

Un tel mode de réalisation, supposant une zone de stockage de grande hauteur, permet de traiter et d'analyser un plus grand nombre de gouttes.

De préférence, le circuit microfluidique est constitué, au moins en partie, d'un matériau transparent permettant de voir au moins l'une des zones de stockage, depuis l'extérieur du circuit.

L'analyse optique des gouttes après leur traitement est ainsi facilitée.

### 5. Liste des figures

La présente invention sera mieux comprise à la lecture de la description suivante de modes de réalisation préférés, donnés à titre illustratif et non limitatif, et accompagnée de figures, parmi lesquelles :
- la figure 1 est un plan, en vue de dessus, d'un circuit microfluidique permettant la mise en oeuvre d'un procédé selon un premier mode de réalisation de l'invention ;
- la figure 2 est une section du circuit microfluidique de la figure 1 ;
- les figures 3A, 4A, 5A et 6A sont des détails du plan de la figure 1, à différents moments de l'utilisation du circuit microfluidique ;
- les figures 3B, 4B, 5B et 6B sont des sections correspondant respectivement aux figures 3A, 4A, 5A et 6A ;
- les figures 7, 8 et 9 sont respectivement un plan et deux sections d'un circuit microfluidique permettant la mise en oeuvre d'un procédé selon un second mode de réalisation possible de l'invention ;
- les figures 10 et 11 sont respectivement un plan et une section d'un circuit microfluidique permettant la mise en oeuvre d'un procédé selon un troisième mode de réalisation possible de l'invention ;
- les figures 12 et 13 sont respectivement un plan et une section d'un circuit microfluidique permettant la mise en oeuvre d'un procédé selon un quatrième mode de réalisation possible de l'invention.

### 6. Description détaillée d'un mode de réalisation

### 6.1. Circuit microfluidique

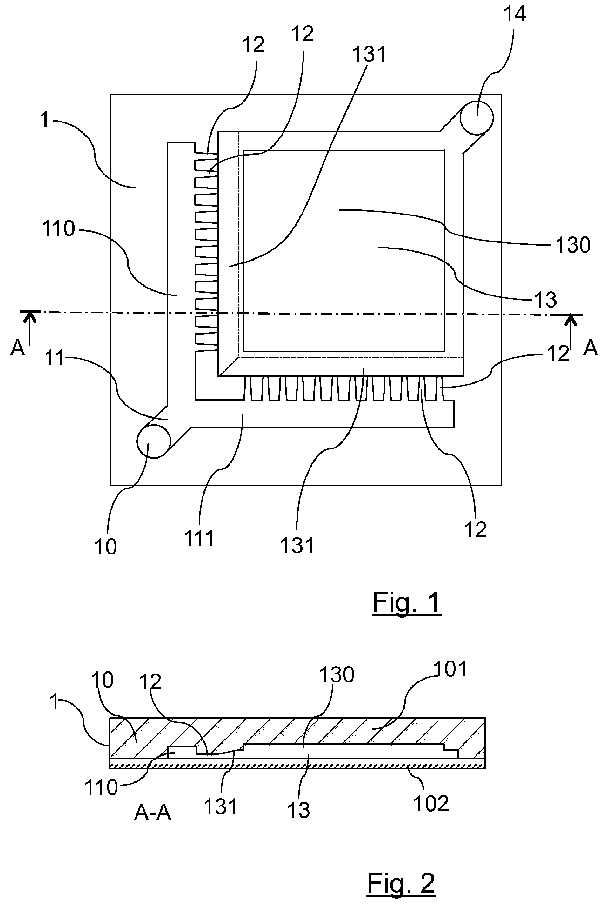

La figure 1 est un plan, en vue de dessus, d'un circuit microfluidique 1 permettant de mettre en oeuvre un procédé selon un mode de réalisation préférentiel de l'invention. Ce plan montre les différents canaux microfluidiques qui sont ménagés à l'intérieur de ce circuit microfluidique. Une section de ce circuit microfluidique 1 est également représentée sur la figure 2.

De façon connue en elle-même, ce circuit microfluidique 1 peut être composé de deux plaques superposées, collées l'une à l'autre. Ainsi, le circuit 1 est composé d'une plaque 102, qui peut par exemple être une lame transparente de microscope, et d'une plaque 101, dont la faces en contact avec la plaque 102 est gravée de façon à définir des microcanaux entre les deux plaques qui sont superposées et collées l'une à l'autre. La plaque 101 peut être constituée d'un matériau polymère. De préférence, le matériau constituant au moins l'une des deux plaques est transparent, afin de faciliter l'observation des fluides dans les microcanaux. Dans ce cas, l'observation du circuit 1 permet de voir les microcanaux par transparence, comme le représente la figure 1.

De façon connue, les dimensions de ces microcanaux peuvent être choisies librement en adaptant la largeur et la profondeur des gravures dans la plaque gravée. Par exemple, les microcanaux peuvent avoir une largeur de 100 µm environ et une profondeur de 50 µm environ. Ces microcanaux peuvent également présenter des dimensions plus importantes, ou au contraire plus faibles, de façon à s'adapter aux caractéristiques de différents fluides, ou aux tailles des gouttes à manipuler. Il est à noter que des circuits microfluidiques fabriqués selon d'autres méthodes connues de l'homme du métier peuvent évidemment être utilisés pour mettre en oeuvre l'invention.

Ces microcanaux sont normalement dimensionnés pour que leurs parois exercent une contrainte confinant la solution ou sur les gouttes qui y circulent. Dans la plupart des microcanaux, les gouttes sont ainsi confinées par les parois supérieure, inférieure, droite et gauche. Certains microcanaux, appelés « chambres » par la suite, sont cependant dimensionnés de façon à n'exercer une contrainte que dans une dimensions, deux de leurs parois sensiblement parallèles (généralement la paroi supérieure et la paroi inférieure) étant proches l'une de l'autre pour confiner les gouttes, et les autres parois étant suffisamment éloignées pour ne pas confiner les gouttes.

Le circuit microfluidique 1 doit, préalablement à son utilisation, être rempli d'un fluide inerte, appelé par la suite fluide porteur, qui n'est pas miscible avec les fluides que l'on souhaite manipuler dans le circuit. Ce fluide porteur est généralement de l'huile, pouvant être additionnée d'un produit additif surfactant permettant d'éviter la fusion spontanée de gouttes de solution manipulées, si elles entrent en contact. Cet additif surfactant peut parfois être inutile, en fonction des caractéristiques de l'huile utilisée comme fluide porteur et de la solution à traiter et analyser.

Le circuit microfluidique 1 représenté comporte un microcanal d'alimentation 11, se divisant en deux branches d'alimentation 110 et 111 s'étendant perpendiculairement l'un à l'autre. Ce microcanal 11 est connecté à un trou d'alimentation 10 qui est percé dans l'une des plaques composant le circuit microfluidique 1, et dans lequel peut être introduite l'aiguille d'une seringue ou l'extrémité d'une pipette afin d'injecter un fluide dans le canal d'alimentation 11.

La chambre 13 présente également une ouverture d'évacuation reliée à un trou 14 percé à travers l'une des plaques du circuit 1. Cette ouverture permet notamment l'évacuation d'une partie du fluide porteur, quand le volume total de fluide inséré dans les microcanaux est supérieur au volume de ces microcanaux.

### 6.2. Formation des gouttes

Les deux branches d'alimentation 110 et 111 sont chacune connectées à une pluralité de buses de formation de gouttes 12. Pour des raisons de clarté, les buses ont été représentées sur la figure 1 avec des dimensions supérieures à leurs dimensions normales. Par ailleurs, seules certaines des buses 12 sont référencées sur la figure 1.

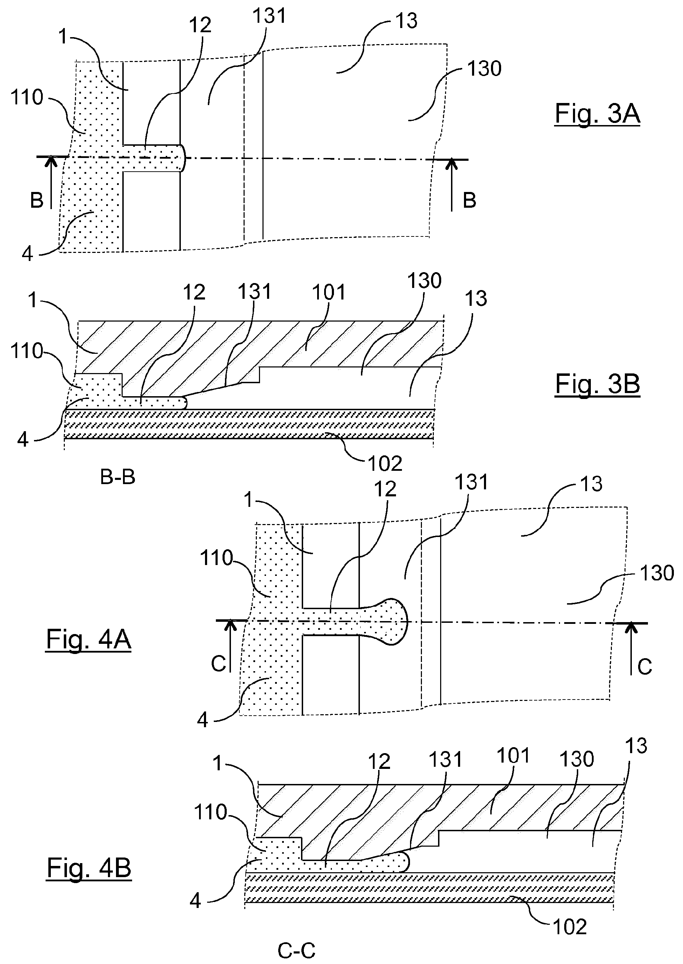

Ces buses de formation de gouttes 12 sont des microcanaux, ou conduites de faible section pouvant être alimentées en fluide par leur première extrémité et faisant passer un faible écoulement de ce fluide vers une deuxième extrémité. Les figures 3A, 4A, 5A et 6A représentent en détail le plan d'une buse de formation de gouttes 12 et de la chambre dans lequel elle débouche, à plusieurs moments de la formation d'une goutte de fluide. Cette buse et cette chambre sont également représentés en détail par les sections des figures 3B, 4B, 5B et 6B, qui correspondent respectivement aux vues des figures 3A, 4A, 5A et 6A. Dans un souci de clarté, le fluide porteur qui remplit les canaux du circuit 1 n'est pas représenté sur ces figures.

Comme le montrent ces figures, la deuxième extrémité de la buse 12 débouche sur une chambre centrale 13, qui présente une surface supérieure gravée dans la plaque 101 et une surface inférieure constituée par la plaque 102. A proximité de la seconde extrémité de la buse 12, la surface supérieure de la chambre 13 présente une zone inclinée 131, de telle sorte que les deux surfaces de la chambre 13 s'écartent quand elles s'éloignent de la seconde extrémité de la buse 12. Cet écartement des parois permet que le confinement subi par la solution diminue au cours de son trajet, après son passage dans la buse 12.

Il est à noter que, selon une variante possible non représentée sur les figures, la zone inclinée peut être remplacée par une zone formant une succession de plusieurs marches dans la surface de la chambre, sans sortir du cadre de l'invention. L'homme du métier sait en effet qu'une telle succession de marches présente le même effet technique qu'une zone inclinée. De même, il serait possible selon d'autres modes de réalisation, que les parois s'écartent en largeur plutôt que de s'écarter en hauteur.

Comme le montrent les figures 3A et 3B, quand un fluide 4, par exemple une solution contenant un matériel biologique, est introduit dans le circuit microfluidique 1 par le trou 10, il remplit la branche d'alimentation 110 et la buse 12. Quand l'introduction du fluide 4 dans le trou 10 se poursuit, le front de l'écoulement de fluide 4 s'avance dans la chambre 13, comme le montrent les figures 4A et 4B. Ce fluide se trouve alors confiné entre une surface inférieure, constituée par la plaque 102, et une surface supérieure, constituée par la zone inclinée 131, qui s'écartent l'une de l'autre en s'éloignant de la buse 12.

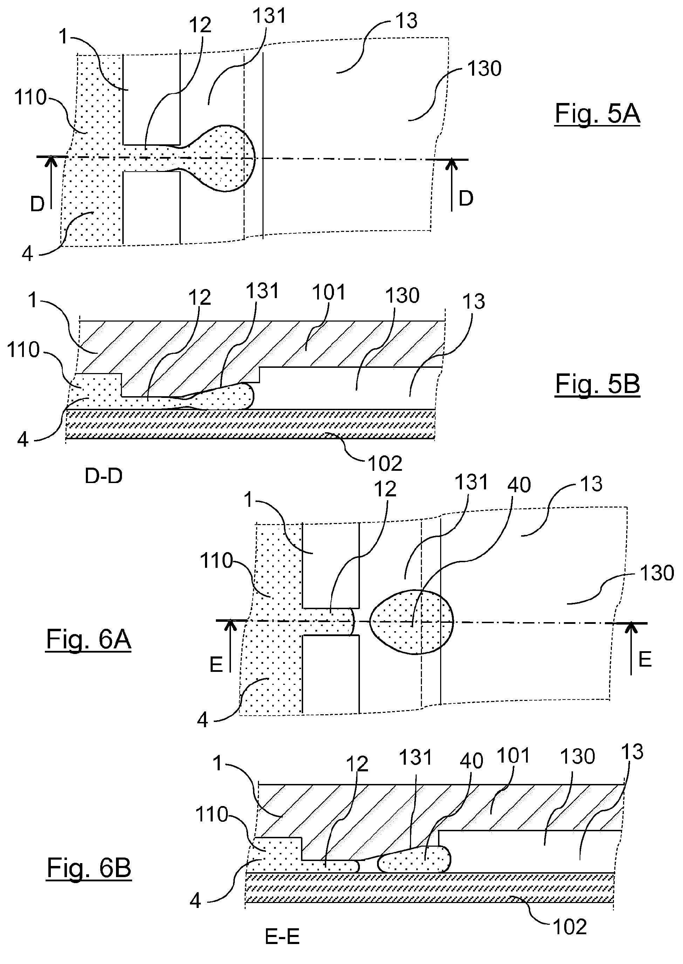

Cet écartement des surfaces tend à attirer le fluide 4 loin de la buse 12. En effet, le fluide tend à prendre une forme la plus proche possible d'une sphère, qui est la forme dans laquelle son énergie de surface est minimale. Il tend donc à se déplacer vers les espaces dans lesquels il est moins confiné. Cette attraction déforme le front de fluide, comme le montrent les figures 5A et 5B, et induit par cette déformation le détachement d'une goutte 40, comme le représentent les figures 6A et 6B, à partir d'une taille critique déterminée par les paramètres géométriques.

Ainsi, la forme des microcanaux du circuit microfluidique 1, et plus précisément la succession d'une buse de formation de goutte 12 et d'une chambre 13 dans laquelle les surfaces s'écartent les unes des autres en s'éloignant de la buse 12, permet la formation de gouttes 40 de fluide 4, sans qu'aucun flux du fluide porteur ne soit nécessaire. La seule action nécessaire pour former ces gouttes est en effet l'introduction du fluide 4 dans le trou 10 avec une pression suffisante.

Alternativement, la formation des gouttes peut aussi être réalisée en appliquant une aspiration (ou une pression négative) à la sortie 14 du circuit microfluidique, après avoir introduit le fluide 4 dans le trou 10. Les gouttes se forment alors de la même manière.

Il est à noter à cet égard que la pression d'introduction du fluide 4 dans le circuit microfluidique 1 n'influe que très faiblement sur la taille des gouttes 40 formées. Il a ainsi été montré par les inventeurs qu'une multiplication par mille de la pression d'introduction du fluide 4 ne multiplie que par deux la taille de la goutte produite. Le circuit microfluidique 1 permet donc de produire des gouttes 40 dont la taille découle principalement des caractéristiques géométriques des microcanaux (et notamment de la section de la buse 12 et de la pente de la zone inclinée 131) et de la viscosité du fluide 4. Chaque buse 12 peut ainsi, quand elle est alimentée en amont par un flux continu de fluide, ici par le fluide provenant des branches d'alimentation 110 et 111, fournir en aval des gouttes de taille homogène de ce même fluide.

De telles buses 12 de formation de gouttes, qui permettent de former un train de gouttes à partir d'un écoulement continu de fluide sans avoir besoin de disposer d'un flux de fluide porteur, sont décrites dans le document WO 2011/121220, au nom des demanderesses.

Vingt-quatre buses 12 sont représentées sur le circuit microfluidique 1 de la figure 1. Il est cependant évident que des buses similaires plus nombreuses, et de taille inférieure, peuvent être mises en oeuvre dans d'autres circuits microfluidiques permettant de mettre en oeuvre l'invention. A titre d'exemple, un circuit microfluidique comportant 256 buses de hauteur 50 µm et de largeur 100 µm chacune, permet de décomposer un échantillon d'environ 20 µl de solution en environ 100 000 gouttes en deux minutes.

Il est à noter que, selon d'autres modes de réalisation possibles, les buses peuvent être réparties autour de trois côtés, ou des quatre côtés d'une chambre rectangulaire, ou être réparties autour d'une partie ou de la totalité de la périphérie d'une chambre présentant une forme différente, par exemple ronde, hexagonale, etc. Ces très nombreuses variantes sont rendues possibles par le mode de production des gouttes sans flux de fluide porteur, qui permet une production simultanée d'un très grand nombre de gouttes sans qu'il soit nécessaire de prévoir la circulation et l'évacuation d'un grand volume de fluide porteur.

### 6.3. Stockage des gouttes

Chacune des buses 12 de formation de gouttes débouchant dans la même chambre 13, l'ensemble des gouttes produites est concentré dans une zone de stockage de cette chambre. Le terme «zone de stockage,», ou «zone de piégeage», désigne dans la présente description une zone du circuit microfluidique dans laquelle une goutte peut pénétrer, mais dont elle ne peut pas sortir sans une intervention extérieure.

Dans le mode de réalisation représenté, une zone est gravée en creux dans la surface supérieure de cette chambre 13, de façon à former une zone de stockage des gouttes 130, situé au milieu de la chambre 13. Autour de la zone de stockage 130, la chambre 13 présente des surfaces supérieure et inférieure qui sont de préférence parallèles et qui sont suffisamment proches pour que les gouttes placées dans la chambre soient confinées entre ces deux surfaces, sans pouvoir prendre la forme sphérique qui correspond à une énergie de surface minimale.

Du fait de la gravure en creux, la distance entre la surface supérieure de la chambre et sa surface inférieure est plus importante (par exemple d'environ 50 µm) dans la zone de stockage que dans les zones voisines. Une goutte placée dans cette zone de stockage peut donc prendre une forme plus compacte qu'une goutte confinée entre les surfaces supérieure et inférieure de la chambre 13, autour de la zone de stockage 130. En conséquence, une goutte se trouvant dans la zone de stockage présente une énergie de surface inférieure à une goutte se trouvant hors de cette zone. Une goutte placée dans cette zone de stockage ne peut donc pas en sortir sans qu'une énergie lui soit apportée pour augmenter son énergie de surface.

Il est à noter que la technique de piégeage des gouttes dans le circuit microfluidique est décrite dans le document WO 2011/039475, au nom des demanderesses.

La zone de stockage 130 forme donc un espace dans lequel les gouttes sont maintenues, et est de préférence dimensionné de telle sorte que les gouttes y soient disposées dans un plan, en deux dimensions. Toute ses gouttes contenues dans cette zone sont ainsi directement visible depuis l'extérieur du circuit microfluidique, du fait de la transparence d'au moins une des surfaces de la chambre.

Il est cependant possible, selon d'autres modes de réalisation, de mettre en oeuvre une zone de stockage dans laquelle la surface supérieure et la surface inférieure sont suffisamment espacées pour recevoir des gouttes réparties sur plusieurs couches.

De préférence, la zone de stockage 130 est située à proximité de l'endroit où les gouttes se forment. Ainsi, les gouttes s'introduisent dans cette zone de stockage 130 dès leur formation, sans qu'aucun moyen extérieur ne soit nécessaire pour les déplacer vers cette zone. La conformation des parois de la chambre 13, et notamment l'écartement des parois au niveau de la zone inclinée 131 et des bords de la zone de stockage 130, permet en effet que chaque goutte se déplace sous l'effet de sa tension de surface jusqu'à cette zone de stockage. Il est également possible que les gouttes se déplacent dans la chambre 13, vers la zone de stockage, en étant poussées par d'autres gouttes.

### 6.4. Traitement et analyse d'une solution dans ce circuit microfluidique

Le circuit microfluidique 1 est, avant son utilisation, rempli par un fluide porteur. Pour réaliser un traitement et une analyse d'une solution contenant un matériel biologique, un opérateur introduit cette solution par le trou d'alimentation 10. Cette introduction se fait simplement en ajustant l'extrémité d'une pipette ou l'aiguille d'une seringue dans le trou 10 avant d'éjecter ce fluide en pressant sur la seringue ou la pipette. Le fluide s'écoule alors dans le canal d'alimentation 11, puis dans ses branches 110 et 111. Il traverse ensuite les différentes buses 12, à la sortie desquelles il se décompose en gouttes qui s'écoulent dans la chambre 13. Du fait du grand nombre de buses 12 réparties le long des branches 110 et 111 du canal d'alimentation, un grand nombre de gouttes peuvent être créées simultanément. Ces gouttes sont captées et retenues dans la zone de stockage 130, et remplissent rapidement l'intégralité de cette zone de stockage.

Il est à noter que la fabrication des gouttes se fait d'une façon particulièrement simple et efficace. En effet l'opérateur doit juste introduire la solution dans un orifice, sans avoir besoin d'équilibrer la vitesse d'écoulement de ce fluide avec la vitesse d'écoulement d'un fluide porteur. Par ailleurs, la pression exercée par l'opérateur sur la seringue ou la pipette, n'a qu'une très faible influence sur la taille des gouttes produites. L'opérateur peut donc injecter la solution dans le trou 10 sans prendre de précaution particulière pour assurer une pression parfaitement constante. Les gouttes qui sont formées par les buses 12, dès le début de la formation des gouttes, présentent de toute façon des dimensions homogènes.

L'opérateur peut surveiller le remplissage de la chambre 13 et cesser d'injecter la solution quand la zone de stockage 130 est complètement remplie, pour éviter que des gouttes de la solution ne s'échappent par l'ouverture d'évacuation reliée au trou 14.

Si le volume d'échantillon permettant de créer suffisamment de gouttes pour remplir la zone de stockage est connu, il est également possible d'injecter précisément ce volume de la solution, pour éviter de perdre une partie de l'échantillon. Dans ce cas, il peut être utile d'injecter une petite quantité de fluide porteur dans le trou 10 après l'injection de solution, afin de repousser la solution restant dans le canal d'alimentation 11 et ses branches 110 et 111 vers la chambre 13.

Lorsque la zone de stockage 130 de la chambre 13 est remplie de gouttes de la solution à traiter et à analyser, l'opérateur peut retirer la pipette ou la seringue du trou 10. Du fait du maintien des gouttes dans la zone de stockage 130, le circuit microfluidique 1 peut alors être manipulé par l'opérateur sans risque d'échappement des gouttes. L'ensemble du circuit microfluidique 1 peut par exemple être placé dans un appareil de chauffage permettant son thermocyclage, ou tout autre traitement thermique, sans risque de perte d'une partie de l'échantillon de solution divisé en gouttes. Il est également possible de réaliser d'autres types de traitement, en plus ou à la place d'un traitement thermique.

Après un traitement, une analyse optique des gouttes peut être réalisée très facilement, toutes les gouttes contenues dans la zone de stockage 130 de la chambre 13 étant avantageusement visibles par une face transparente du circuit microfluidique 1. Cette analyse peut, avantageusement, être réalisée de manière automatisée.

### 6.5. Modes de réalisation avec plusieurs zones de stockage

Un grand nombre de variantes de ce procédé peuvent être mis en oeuvre sans sortir du cadre de l'invention, notamment en utilisant des circuits microfluidiques spécialement conçus pour s'adapter à des conditions d'expérimentations variées.

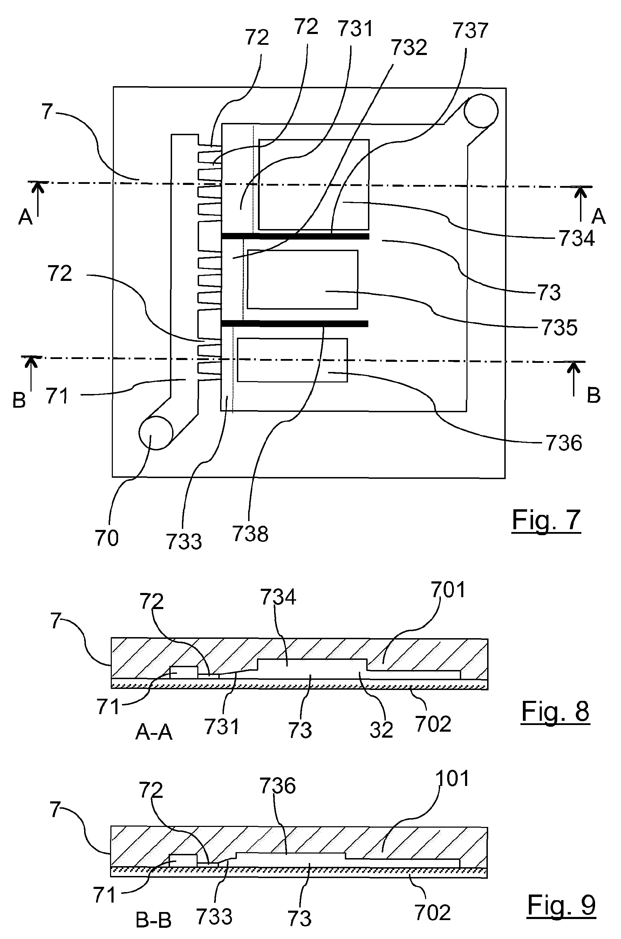

Ainsi, la figure 7 est un plan, en vue de dessus, d'un circuit microfluidique 7 permettant de mettre en oeuvre un procédé selon un second mode de réalisation possible de l'invention. Des sections de ce circuit microfluidique 7 sont également représentées par les figures 8 et 9. Comme le circuit microfluidique 1, le circuit microfluidique 7 est composé d'une plaque 702 transparente et d'une plaque 701 gravée de façon à définir des microcanaux entre les deux plaques, quant elles sont superposées et collées l'une à l'autre.

Ce circuit microfluidique 7 comporte un trou d'alimentation 70 connecté à un microcanal d'alimentation 71. Douze buses de formation de gouttes 72 sont connectées à ce microcanal d'alimentation 71, et débouchent sur une chambre 73. Dans le mode de réalisation représenté, toutes les buses 72 (qui, pour des raisons de clarté, ne sont pas toutes référencées sur la figure 7) sont identiques. Elles sont de préférence du même type que les buses 12 du circuit microfluidique 1.

Dans ce mode de réalisation, la surface supérieure de la chambre 73 présente plusieurs zones inclinées, respectivement 731, 732 et 733, présentant des pentes différentes. Chacune de ces zones inclinées est située à proximité de l'extrémité de certaines des buses 72. Ainsi, la zone inclinée 731, visible notamment sur la section de la figure 8, présente une pente relativement faible, de telle sorte que les surfaces supérieures et inférieures de la chambre 73 s'écartent faiblement l'une de l'autre quand elles s'éloignent des buses 72. Au contraire, la zone inclinée 733, visible notamment sur la section de la figure 9, présente une pente relativement forte, de telle sorte que les surfaces inférieures et supérieures de la chambre 73 s'écartent fortement en s'éloignant des buses 72. La zone inclinée 732 présente une pente intermédiaire.

Du fait des pentes différentes, les gouttes qui sont produites par les buses 72 et les surfaces de la chambre 73 sont de tailles différentes pour chacune des zones inclinées. Ainsi, les gouttes produites au niveau de la zone inclinée 731 sont plus grandes que celles produites au niveau de la zone inclinée 732, elle même plus grande que celle produites au niveau de la zone inclinée 733.

Trois zones de stockage des gouttes sont définies par gravure dans la surface supérieure de la chambre 73. La zone de stockage 734 est située à proximité de la zone inclinée 731 afin de recueillir les gouttes formées au niveau de cette zone inclinée. De même, les zones de stockage 735 et 736 sont placées, respectivement, à proximité des zones inclinées 732 et 733. De façon avantageuse, les dimensions de chacune de ces zones de stockage sont adaptées aux dimensions et à la quantité des gouttes qu'elles sont destinées à recevoir.

Dans le mode de réalisation représenté, des cloisons 737 et 738, s'élevant sur toute la hauteur de la chambre 73, permettent de cloisonner partiellement celle-ci pour éviter que certaines des gouttes se déplacent vers une zone de stockage qui ne leur est pas destinée.

Ainsi, le circuit microfluidique 7 permet de préparer, de façon simultanée, des échantillons de gouttes de tailles différentes d'une même solution. Ces échantillons peuvent alors subir les mêmes traitements, avant d'être analysés. Un tel procédé peut être utile, par exemple, pour l'analyse d'une solution pour laquelle la dimension de gouttes permettant d'obtenir un résultat optimal n'est pas connue.

Bien entendu, l'homme du métier peut facilement mettre en oeuvre des variantes de ce mode de réalisation, par exemple en utilisant des buses de formation de gouttes de tailles différentes débouchant sur une même zone inclinée, sans sortie du cadre de la présente invention.

### 6.6 Circuit microfluidique avec plusieurs zones de stockage identiques.

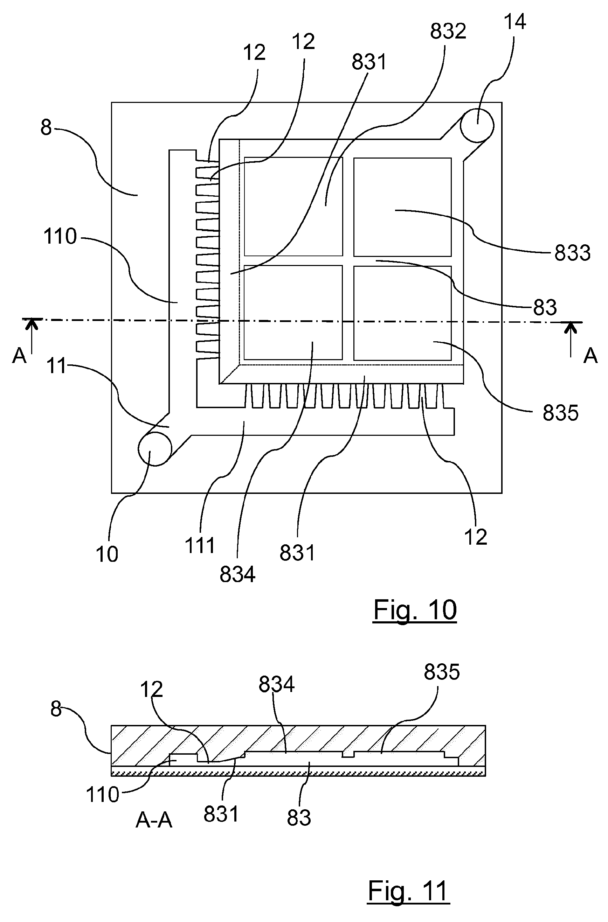

La figure 10 est un plan, en vue de dessus, d'un circuit microfluidique 8 permettant de mettre en oeuvre un procédé selon un troisième mode de réalisation possible de l'invention. Une section de ce circuit microfluidique 8 est également représentée par la figure 11. Ce circuit 8 est en grande partie identique au circuit microfluidique 1. Il comporte notamment le même trou d'alimentation 10, le même microcanal d'alimentation 11 se divisant en deux branches d'alimentation 110 et 111, et les mêmes buses de formation de gouttes 12. La chambre centrale 83, dans laquelle débouchent les buses de formation de gouttes 12, présente des zones inclinées 831 identiques aux zones inclinées 131 du circuit microfluidique 1.

Dans ce mode de réalisation, la paroi supérieure de la chambre 83 est gravée de façon à définir, non pas une, mais quatre zones de stockage des gouttes distinctes. Ces quatre zones de stockage 832, 833, 834 et 835 sont, dans le mode de réalisation représenté, identiques. Elles peuvent cependant, pour les besoins d'une expérience, présenter des dimensions différentes, par exemple pour contenir des gouttes réparties en un nombre de couches différent.

Lors de la production des gouttes, celles-ci remplissent les différentes zones de stockage, le cas échéant en étant poussées vers ces zones de stockage par d'autres gouttes.

### 6.7 Circuit micro fluidique avec piégeage individuel des gouttes.

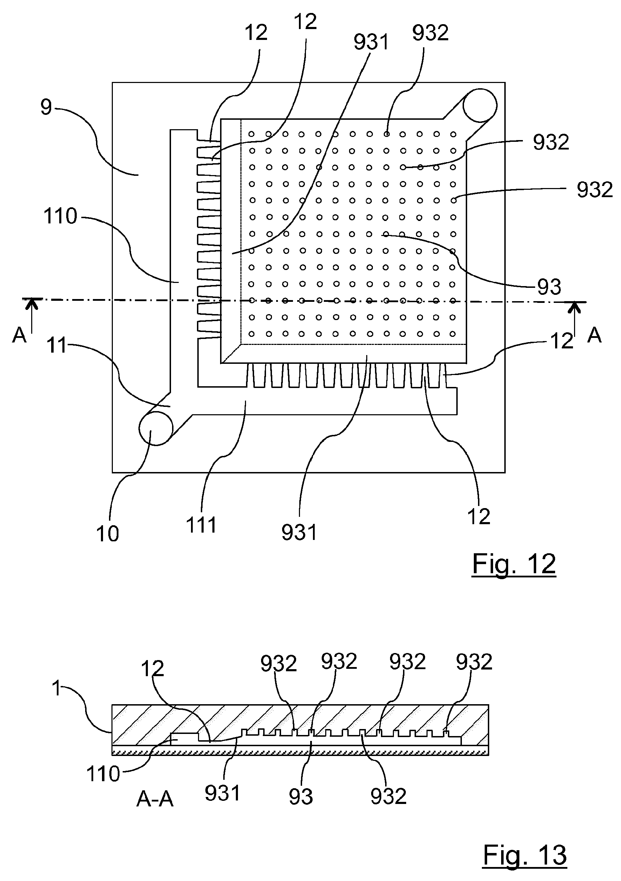

La figure 12 est un plan, en vue de dessus d'un circuit microfluidique 9 permettant de mettre en oeuvre un procédé selon un quatrième mode de réalisation possible de l'invention. Une section de ce circuit microfluidique 9 est également représenté par la figure 13. Ce circuit 9 est également en grande partie identique au circuit microfluidique 1. Il comporte notamment le même trou d'alimentation 10, le même microcanal d'alimentation 11 se divisant en deux branches d'alimentation 110 et 111, et les mêmes buses de formation de gouttes 12. La chambre centrale 93, dans laquelle débouchent les buses de formation de gouttes 12, présente des zones inclinées 931 identiques aux zones inclinées 131 du circuit microfluidique 1.

La paroi supérieure de cette chambre 93 est gravée de façon à définir une pluralité de petits trous 932. Ces trous 932 (qui ne sont pas tous référencés sur les figures 12 et 13, pour des raisons de clarté) peuvent présenter un diamètre faible, par exemple compris entre environ 10% et 120% du diamètre d'une goutte. Chacun de ces trous 932 constitue une zone de stockage, ou un « piège », capable de recevoir une seule goutte.

Lorsque les gouttes se formant remplissent la chambre 93, elles se placent sur chacune de ces zones de stockage 932, le cas échéant en étant poussée d'une zone de stockage vers une autre par une autre goutte. Il est également possible, selon une variante de ce mode de réalisation, que la paroi supérieure de la chambre 93 ne soit pas parfaitement parallèle à sa paroi inférieure, afin de former une légère pente favorisant le déplacement des gouttes vers les zones de stockage 932 qui sont les plus éloignées des buses de formation de gouttes 12.

Chacune des zones de stockage 932 est ainsi rapidement occupée par une goutte unique. Le circuit microfluidique 9 permet ainsi de produire, de traiter et d'analyser une pluralité de gouttes qui occupent chacune une position bien précise, connue à l'avance. Une telle disposition des gouttes peut faciliter considérablement l'analyse optique des résultats d'un traitement effectué sur les gouttes.

Par ailleurs, dans ce mode de réalisation, les gouttes produites ne restent pas en contact prolongé les unes avec les autres. En effet, les positions des différentes zones de stockage 932 sont avantageusement choisies pour que les gouttes piégées ne se touchent pas. Cette absence de contact prolongé entre les gouttes réduit considérablement le risque de fusion de plusieurs gouttes en une seule. En conséquence, dans ce mode de réalisation l'usage d'un additif surfactant (tensioactifs utilisés pour éviter la coalescence de gouttes entre elles), additionné au fluide porteur, peut s'avérer inutile. Dans d'autres cas, un additif surfactant peu performant peut être suffisant. Ce mode de réalisation est donc particulièrement avantageux en ce qu'il permet d'éviter l'utilisation d'additifs surfactants les plus performants, qui peuvent être coûteux.

### 6.8. Avantages de l'invention par rapport aux solutions antérieures

Le procédé selon l'invention permet donc de rendre plus rapide, plus efficace, plus simple et moins couteux le traitement et l'analyse d'une solution contenant un matériel biologique divisée en gouttes.

En effet, il permet de simplifier au maximum la préparation de l'échantillon à traiter. Il suffit que l'opérateur injecte dans un circuit microfluidique adapté la solution à analyser, sans se préoccuper de la pression d'injection, pour que cette solution soit divisée en gouttes confinées dans le circuit, prêtes à subir un traitement thermique et à être analysées. Par ailleurs, la manipulation du circuit contenant les gouttes ne nécessite aucune précaution particulière.

Cette solution est donc plus simple, plus rapide et moins couteuse à mettre en oeuvre que les solutions de l'art antérieur nécessitant l'équilibrage de deux écoulements de fluides pour la production des gouttes de solution.

De plus, le procédé selon l'invention permet que la quasi-totalité de la solution consommée soit divisée en gouttes pouvant être traitées et analysée, ce qui est avantageux par rapport aux solutions de l'art antérieur qui induisent la perte d'une part significative de la solution traitée.

Enfin, le procédé selon l'invention permettant de fabriquer les gouttes sans mettre en oeuvre d'écoulement de fluide porteur, les buses de formation de gouttes peuvent être réparties sur plusieurs côtés de la chambre destinée à recueillir les gouttes. Il est ainsi possible de les répartir sur deux côtés d'une chambre carrée, comme le représente par exemple le mode de réalisation de la figure 1. Il est également possible de les répartir sur trois ou quatre côtés d'une telle chambre. Il est également possible de les répartir autour d'une chambre de forme différente, par exemple autour de la quasi-totalité du diamètre d'une chambre circulaire.

Cette répartition possible d'un grand nombre de buses de formation de gouttes autour d'une chambre, qui n'est pas possible avec les solutions de l'art antérieur dans lesquelles la fabrication des gouttes s'accompagne d'un flux de fluide porteur, qui doit pouvoir s'échapper, permet une grande efficacité dans la fabrication des gouttes. Le procédé selon l'invention permet notamment de réaliser, de façon plus rapide, plus efficace, plus simple et moins couteuse qu'avec les procédés de l'art antérieur, une PCR digitale par gouttes.

Ce procédé permet également de réaliser d'autres types de traitement et d'analyse de solutions contenant un matériel biologique. Ainsi, il est par exemple possible d'introduire dans le circuit microfluidique une solution contenant une faible quantité d'enzymes et un substrat capable de réagir avec l'enzyme. Un certain temps après la formation des gouttes, il est possible de les analyser optiquement (soit de façon automatique, soit par une observation visuelle et un comptage) pour déterminer la proportion des gouttes dans lesquelles une réaction enzymatique s'est produite, et quantifier ainsi la présence d'enzyme. Dans cet exemple, le traitement appliqué aux gouttes est une incubation, consistant uniquement à les maintenir pendant un temps suffisamment long à des conditions de température permettant la réaction enzymatique.

Il est également possible, par exemple, d'introduire dans le circuit microfluidique une solution contenant des cellules et des marqueurs capables d'interagir avec certaines de ces cellules. Un certain temps après la formation des gouttes, il est possible de les analyser optiquement (soit de façon automatique, soit par une observation visuelle et un comptage) pour déterminer la proportion des gouttes dans lesquelles des cellules ont interagi avec les marqueurs, et quantifier ainsi la présence des cellules à caractériser. Là encore, le traitement appliqué aux gouttes est une simple incubation.

Enfin, le circuit microfluidique objet de l'invention, permettant de mettre en oeuvre le procédé selon l'invention, est lui-même particulièrement simple et peu couteux à fabriquer. De nombreuses variantes de ce circuit peuvent être mises en oeuvre facilement. Il est ainsi possible, par exemple que la chambre centrale du circuit constitue elle-même la zone de stockage des gouttes, à condition que des moyens adaptés empêchent les gouttes d'en sortir sans intervention extérieure.

## Revendications

1. Procédé microfluidique de traitement et d'analyse d'une solution contenant un matériel biologique, **caractérisé en ce que** ledit procédé comprend les étapes suivantes :
- introduction de ladite solution dans des microcanaux d'un circuit microfluidique (1, 7, 8, 9) ;
- détachement de gouttes (40) de ladite solution dans un fluide porteur, causé par écartement des parois desdits microcanaux, couplé avec les effets de la tension de surface de ladite solution ;
- déplacement d'au moins une partie desdites gouttes (40) dans ledit fluide porteur vers au moins une zone de stockage (130, 734, 735, 736, 832, 833, 834, 835, 932) de gouttes dans ledit circuit microfluidique (1, 7, 8, 9), causé par écartement des parois desdits microcanaux, couplé avec les effets de la tension de surface desdites gouttes ;
- concentration desdites gouttes de ladite partie desdites gouttes déplacées dans ladite zone de stockage, les dimensions de ladite zone de stockage étant adaptées aux dimensions et à la quantité de gouttes contenues dans ladite partie desdites gouttes déplacées,
- application d'un traitement auxdites gouttes (40) situées dans ladite ou lesdites zones de stockage (130, 734, 735, 736, 832, 833, 834, 835, 932);
- analyse desdites gouttes (40) situées dans ladite ou lesdites zones de stockage (130, 734, 735, 736, 832, 833, 834, 835, 932).

2. Procédé microfluidique selon la revendication 1, **caractérisé en ce que** ledit fluide porteur dans lequel lesdites gouttes (40) sont détachées et déplacées est sensiblement statique.

3. Procédé microfluidique selon l'une quelconque des revendications précédentes, **caractérisé en ce que** ledit traitement appliqué auxdites gouttes (40) comprend des variations de la température des gouttes.

4. Procédé microfluidique selon la revendication précédente, **caractérisé en ce que** lesdites variations de température sont appliquées à l'ensemble du circuit microfluidique contenant lesdites gouttes (40).

5. Procédé microfluidique selon l'une quelconque des revendications précédentes, **caractérisé en ce que** ladite analyse desdites gouttes (40) est une analyse optique.

6. Procédé microfluidique selon l'une quelconque des revendications précédentes, **caractérisé en ce qu'**au moins une desdites zones de stockage (130, 734, 735, 736, 832, 833, 834, 835, 932) est constituée par une zone dans laquelle lesdites les gouttes (40) présentent une énergie de surface plus faible que dans les zones voisines.

7. Procédé microfluidique selon l'une quelconque des revendications précédentes, **caractérisé en ce que** ledit matériel biologique contenu dans ladite solution comprend au moins un acide nucléique, et ledit traitement appliqué aux gouttes est une amplification en chaîne par polymérase, permettant d'augmenter la concentration d'au moins une séquence dudit acide nucléique.

8. Circuit microfluidique (1, 7, 8, 9), pour la mise en oeuvre du procédé selon l'une quelconque des revendications **1** à **7,** dans lequel sont définis des microcanaux pouvant contenir des fluides, ledit circuit comprenant au moins un dispositif de formation de gouttes d'une solution dans un fluide porteur, et au moins une zone de stockage des gouttes (130, 734, 735, 736, 832, 833, 834, 835, 932) ainsi produites ;
**caractérisé en ce que** lesdits dispositifs de formation des gouttes comprennent des portions de paroi (131, 731, 831, 931) desdits microcanaux, s'écartant les unes des autres de façon à détacher une goutte (40) de ladite solution sous l'effet de la tension de surface de ladite solution ;
et **en ce qu'**il comprend des moyens de guidage des gouttes comprenant des portions de paroi (131, 731, 831, 931) desdits microcanaux, s'écartant les unes des autres de façon à déplacer au moins une partie desdites gouttes (40) vers ladite zone de stockage (130, 734, 735, 736, 832, 833, 834, 835, 932) sous l'effet de la tension de surface desdites gouttes (40),
et **en ce que** les dimensions de ladite zone de stockage sont adaptées aux dimensions et à la quantité de gouttes contenues dans ladite partie desdites gouttes déplacées de façon à concentrer lesdites gouttes de ladite partie desdites gouttes déplacées dans ladite zone de stockage.

9. Circuit microfluidique (1, 7, 8, 9) selon la revendication précédente, **caractérisé en ce qu'**au moins une desdites zones de stockage (130, 734, 735, 736, 832, 833, 834, 835, 932) est constituée par une zone d'un microcanal dans laquelle les parois dudit microcanal sont plus éloignées les unes des autres que dans les zones voisines.

10. Circuit micro fluidique (7, 8, 9) selon l'une quelconque des revendications 8 et 9, **caractérisé en ce que** ledit circuit microfluidique contient au moins deux zones de stockage (734, 735, 736, 832, 833, 834, 835, 932) distinctes.

11. Circuit microfluidique (7) selon l'une quelconque des revendications 8 et 9, **caractérisé en ce que** ledit circuit microfluidique (7) comprend au moins deux dispositifs de formation de gouttes comprennent des buses de section distinctes formant chacun des gouttes de volumes différents.

12. Circuit microfluidique (7) selon l'une quelconque des revendications **8** et **9, caractérisé en ce que** lesdits moyens de guidage des gouttes (731, 732, 733) présentent plusieurs zones d'inclinaison distinctes guidant les gouttes de volumes différents vers des zones de stockage distinctes (734, 735, 736).

13. Circuit microfluidique (9) selon l'une quelconque des revendications précédentes, **caractérisé en ce qu'**au moins une desdites zones de stockage (932) correspond à un trou dont le diamètre est compris entre 10% et 120% du diamètre d'une goutte, ledit trou étant apte à ne recevoir qu'une goutte (40).

14. Circuit microfluidique (1, 7, 8) selon l'une quelconque des revendications **8** à **12, caractérisé en ce qu'**au moins une desdites zones de stockage (130, 734, 735, 736, 832, 833, 834, 835) présente un dimensionnement prédéterminé apte à recevoir des gouttes dans un même plan.

15. Circuit microfluidique (1, 7, 8) selon l'une quelconque des revendications **8** à **12, caractérisé en ce qu'**au moins une desdites zones de stockage (130, 734, 735, 736, 832, 833, 834, 835) présente une distance prédéterminée entre une surface supérieure et une surface inférieure la définissant, ladite distance prédéterminée de ladite au moins une zone de stockage répartissant sur au moins deux plans superposés les gouttes que ladite au moins une zone de stockage contient.

16. Circuit microfluidique (1, 7, 8, 9) selon l'une quelconque des revendications **8** à **15, caractérisé en ce qu'**il est constitué au moins en partie, d'un matériau transparent permettant de voir au moins l'une desdites zones de stockage (130, 734, 735, 736, 832, 833, 834, 835, 932), depuis l'extérieur dudit circuit.

## Patentansprüche

1. Mikrofluidisches Verfahren zum Behandeln und Analysieren einer Lösung, die ein biologisches Material enthält, **dadurch gekennzeichnet, dass** das besagte Verfahren die folgenden Schritte umfasst:
- Einbringen der besagten Lösung in Mikrokanäle einer mikrofluidischen Schaltung (1, 7, 8, 9);
- Loslösen von Tropfen (40) der besagten Lösung in einer Trägerflüssigkeit, verursacht durch das Entfernen der Wandungen von den besagten Mikrokanälen zusammen mit den Auswirkungen der Oberflächenspannung der besagten Lösung;
- Bewegen zumindest eines Teils der besagten Tropfen (40) in der besagten Trägerflüssigkeit in zumindest eine Sammelzone (130, 734, 735, 736, 832, 833, 834, 835, 932) für Tropfen in der besagten mikrofluidischen Schaltung (1, 7, 8, 9), verursacht durch das Entfernen der Wandungen von den besagten Mikrokanälen zusammen mit den Auswirkungen der Oberflächenspannung der besagten Tropfen;
- Konzentrieren der besagten Tropfen des besagten Teils der besagten Tropfen, die in die besagte Sammelzone bewegt werden, wobei die Abmessungen der besagten Sammelzone den Abmessungen und der Menge an Tropfen angepasst sind, die im besagten Teil der besagten bewegten Tropfen enthalten sind,
- Anwenden einer Behandlung auf die besagten Tropfen (40), die sich in der besagten oder in den besagten Sammelzonen (130, 734, 735, 736, 832, 833, 834, 835, 932) befinden;
- Analyse der besagten Tropfen (40), die sich in der besagten oder in den besagten Sammelzonen (130, 734, 735, 736, 832, 833, 834, 835, 932) befinden.

2. Mikrofluidisches Verfahren nach Anspruch 1, **dadurch gekennzeichnet, dass** die besagte Trägerflüssigkeit, in der die besagten Tropfen (40) losgelöst und bewegt werden, in etwa statisch ist.

3. Mikrofluidisches Verfahren nach irgendeinem der vorherigen Ansprüche, **dadurch gekennzeichnet, dass** die besagte Behandlung auf die besagten Tropfen (40) angewandte Variationen der Temperatur der Tropfen umfasst.

4. Mikrofluidisches Verfahren nach dem vorherigen Anspruch, **dadurch gekennzeichnet, dass** die besagten Temperaturvariationen auf die gesamte mikrofluidischen Schaltung angewandt werden, in der die Tropfen (40) enthalten sind.

5. Mikrofluidisches Verfahren nach irgendeinem der vorherigen Ansprüche, **dadurch gekennzeichnet, dass** die besagte Analyse der besagten Tropfen (40) eine optische Analyse ist.

6. Mikrofluidisches Verfahren nach irgendeinem der vorherigen Ansprüche, **dadurch gekennzeichnet, dass** zumindest eine der besagten Sammelzonen (130, 734, 735, 736, 832, 833, 834, 835, 932) durch eine Zone gebildet wird, in der die besagten Tropfen (40) eine Oberflächenenergie aufweisen, die geringer ist, als in den benachbarten Zonen.

7. Mikrofluidisches Verfahren nach irgendeinem der vorherigen Ansprüche, **dadurch gekennzeichnet, dass** das besagte biologische Material, das in der Lösung enthalten ist, zumindest eine Nukleinsäure umfasst, und die besagte Behandlung, die ewandt wird, eine Polymerase-Kettenreaktion ist, die es ermöauf die Tropfen angglicht, die Konzentration von zumindest einer Sequenz der besagten Nukleinsäure zu erhöhen.

8. Mikrofluidische Schaltung (1, 7, 8, 9) zur Anwendung des Verfahrens nach irgendeinem der Ansprüche 1 bis 7, in der Mikrokanäle definiert sind, die Flüssigkeiten enthalten können, wobei die besagte Schaltung zumindest eine Vorrichtung zur Bildung von Tropfen einer Lösung in einer Trägerflüssigkeit umfasst, und zumindest eine Sammelzone für die Tropfen (130, 734, 735, 736, 832, 833, 834, 835, 932), die so erzeugt werden;
**dadurch gekennzeichnet, dass** die besagten Vorrichtungen zur Bildung von Tropfen Wandungsabschnitte (131, 731, 831, 931) der besagten Mikrokanäle umfassen, die sich voneinander entfernen, sodass ein Tropfen (40) der besagten Lösung unter der Wirkung der Oberflächenspannung der besagten Lösung losgelöst wird;
und dadurch, dass sie Mittel zum Leiten der Tropfen umfasst, die Wandungsabschnitte (131, 731, 831, 931) der besagten Mikrokanäle umfassen, die sich voneinander entfernen, sodass zumindest ein Teil der besagten Tropfen (40) durch die Wirkung der Oberflächenspannung der besagten Tropfen (40) in die besagte Sammelzone (130, 734, 735, 736, 832, 833, 834, 835, 932) bewegt wird,
und dadurch, dass die Abmessungen der besagten Sammelzone den Abmessungen und der Menge an Tropfen angepasst sind, die im besagten Teil der besagten bewegten Tropfen enthalten sind, sodass die besagten Tropfen des besagten Teils der besagten Tropfen, die in die besagte Sammelzone bewegt werden, konzentriert werden.

9. Mikrofluidische Schaltung (1, 7, 8, 9) nach dem vorherigen Anspruch, **dadurch gekennzeichnet, dass** zumindest eine der besagten Sammelzonen (130, 734, 735, 736, 832, 833, 834, 835, 932) durch eine Zone eines Mikrokanals gebildet wird, in der die Wandungen des besagten Mikrokanals weiter voneinander entfernt sind, als in den benachbarten Zonen.

10. Mikrofluidische Schaltung (7, 8, 9) nach irgendeinem der Ansprüche **8** und **9, dadurch gekennzeichnet, dass** die besagte mikrofluidische Schaltung zumindest zwei getrennte Sammelzonen (734, 735, 736, 832, 833, 834, 835, 932) enthält.

11. Mikrofluidische Schaltung (7) nach irgendeinem der Ansprüche 8 und 9, **dadurch gekennzeichnet, dass** die besagte mikrofluidische Schaltung (7) zumindest zwei Vorrichtungen zur Bildung von Tropfen umfasst, die Düsen mit unterschiedlichen Querschnitten umfassen, die jeweils Tropfen mit unterschiedlichen Volumina bilden.

12. Mikrofluidische Schaltung (7) nach irgendeinem der Ansprüche 8 und 9, **dadurch gekennzeichnet, dass** die besagten Mittel zum Leiten der Tropfen (731, 732, 733) mehrere unterschiedliche Neigungszonen aufweisen, welche die Tropfen mit unterschiedlichen Volumina in unterschiedliche Sammelzonen (734, 735, 736) leiten.

13. Mikrofluidische Schaltung (9) nach irgendeinem der vorherigen Ansprüche, **dadurch gekennzeichnet, dass** zumindest eine der besagten Sammelzonen (932) einem Loch entspricht, dessen Durchmesser in 10% bis 120% des Durchmessers eines Tropfens enthalten ist, wobei das besagte Loch imstande ist, nur einen Tropfen (40) aufzunehmen.

14. Mikrofluidische Schaltung (1, 7, 8) nach irgendeinem der Ansprüche **8** bis **12, dadurch gekennzeichnet, dass** zumindest eine der besagten Sammelzonen (130, 734, 735, 736, 832, 833, 834, 835) eine vorbestimmte Abmessung aufweist, die imstande ist, Tropfen auf einer selben Ebene aufzunehmen.

15. Mikrofluidische Schaltung (1, 7, 8) nach irgendeinem der Ansprüche **8** bis **12, dadurch gekennzeichnet, dass** zumindest eine der besagten Sammelzonen (130, 734, 735, 736, 832, 833, 834, 835) einen vorbestimmten Abstand zwischen einer oberen Fläche und einer unteren Fläche aufweist, durch die sie definiert wird, wobei der besagte vorbestimmte Abstand der besagten zumindest einen Sammelzone die Tropfen, die die besagte zumindest eine Sammelzone enthält, auf zumindest zwei überlagerte Ebenen verteilt.

16. Mikrofluidische Schaltung (1, 7, 8, 9) nach irgendeinem der Ansprüche **8** bis **15, dadurch gekennzeichnet, dass** sie zumindest teilweise aus einem transparenten Material gefertigt ist, das es ermöglicht, zumindest eine der besagten Sammelzonen (130, 734, 735, 736, 832, 833, 834, 835, 932) von außerhalb der besagten Schaltung aus zu sehen.

## Claims

1. Microfluidic process for treating and analysing a solution containing a biological material, **characterised in that** said process comprises the following steps:
- introducing said solution into microchannels of a microfluidic circuit (1, 7, 8, 9);
- detaching drops (40) of said solution in a carrier fluid, caused by the divergence of the microchannel walls, coupled with the effects of the surface tension of said solution;
- moving at least a portion of said drops (40) in said carrier fluid to at least one drop storage zone (130, 734, 735, 736, 832, 833, 834, 932) in said microfluidic circuit (1, 7, 8, 9), caused by the divergence of said microchannel walls, coupled with the effects of the surface tension of said drops;
- concentrating said drops of said at least a portion of said drops moved in said storage zone ; the dimensions of said storage zone being adapted to the dimensions and the quantity of the drops contained in said portion of said moved drops;
- applying a treatment to said drops (40) situated in said storage zone(s) (130, 734, 735, 736, 832, 833, 834, 932);
- analysing said drops (40) situated in the storage zone(s) (130, 734, 735, 736, 832, 833, 834, 932).

2. Microfluidic process according to claim 1, **characterised in that** said carrier fluid wherein said drops (40) are detached and moved is substantially static.

3. Microfluidic process according to any of the above claims, **characterised in that** said treatment applied to said drops (40) comprises variations of the temperature of the drops.

4. Microfluidic process according to the above claim, **characterised in that** said temperature variations are applied to the entire microfluidic circuit containing said drops (40).

5. Microfluidic process according to any of the above claims, **characterised in that** said analysis of said drops (40) is an optical analysis.

6. Microfluidic process according to any of the above claims, **characterised in that** at least one of said storage zones (130, 734, 735, 736, 832, 833, 835, 932) consists of a zone wherein the drops (40) have a lower surface energy than in the adjacent zones.

7. Microfluidic process according to any of the above claims, **characterised in that** said biological material contained in said solution comprises at least one nucleic acid, and said treatment applied to the drops is a polymerase chain reaction amplification, suitable for increasing the concentration of at least one sequence of said nucleic acid.

8. Microfluidic circuit (1, 7, 8, 9), for implementing the process according to any of the claims **1** to **7,** wherein microchannels suitable for containing fluids are defined, said circuit comprising at least one device for forming drops of a solution in a carrier fluid, and at least one zone (130, 734, 735, 736, 832, 833, 835, 932) for storing the drops produced;
**characterised in that** said devices for forming drops comprise wall portions (131, 731, 831, 931) of said microchannels, diverging so as to detach a drop (40) of said solution under the effect of the surface tension of said solution;
and **in that** it comprises means for guiding the drops comprising wall portions (131, 731, 831, 931) of said microchannels, diverging so as to move at least a portion of said drops (40) to the storage zone (130, 734, 735, 736, 832, 833, 835, 932) under the effect of the surface tension of said drops (40),
and **in that** the dimensions of said storage zones are adapted to the dimensions and the quantity of the drops contained in said portion of said moved drops in order to concentrate said drops of said at least portion of said drops moved in said storage zone.

9. Microfluidic circuit (1, 7, 8, 9) according to the above claim, **characterised in that** at least one of said storage zones (130, 734, 735, 736, 832, 833, 835, 932) consists of a zone of a microchannel wherein the walls of said microchannel are further from each other than in the adjacent zones.

10. Microfluidic circuit (7, 8, 9) according to any of claims **8** and **9, characterised in that** said microfluidic circuit contains at least two separate storage zones (734, 735, 736, 832, 833, 835, 932).

11. Microfluidic circuit (7) according to any of claims **8** and **9, characterised in that** said microfluidic circuit (7) comprises at least two devices for forming drops comprising drop-forming nozzles with different sections each being suitable for forming drops of different volumes.

12. Microfluidic circuit (7) according to any of claims **8** or **9, characterised in that** said means for guiding the drops (731, 732, 733) comprises different inclined zones guiding the drops of different volumes to separate storage zones (734, 735, 736).

13. Microfluidic circuit (9) according to any of the above claims, **characterised in that** at least one of said storage zones (932) is a hole the diameter of which being comprised between 10% and 120% of the diameter of a drop, said hole being adapted to only receive one drop (40).

14. Microfluidic circuit (1, 7, 8) according to any of claims **8** to **12, characterised in that** at least one of the storage zones (130, 734, 735, 736, 832, 834, 835) comprises predetermined dimensions adapted to receive drops in the same layer.

15. Microfluidic circuit (1, 7, 8) according to any of claims **8** to **12, characterised in that** at least one of the storage zones (130, 734, 735, 736, 832, 834, 835) comprises a predetermined distance between the upper surface and the lower surface, said predetermined distance of said at least one storage zone distributing the drops contained in said at least one storage zone on at least two superimposed layers.

16. Microfluidic circuit (1, 7, 8, 9) according to any of claims **8** to **15, characterised in that** it consists, at least in part, of a transparent material suitable for viewing at least one of the storage zones (130, 734, 735, 736, 832, 834, 835, 932), from outside the circuit.
